# EUROPEAN PATENT APPLICATION

(11) **EP 4 487 887 A1**
(43) Date of publication of application: **08.01.2025**
(21) Application number: 23183051.4
(22) Date of filing: 03.07.2023
(51) Int. Cl.: A61M 5/20, A61M 5/32

(54) **DRIVE SPRING AND NEEDLE COVER SPRING FOR AN INJECTION DEVICE**

(71) Applicant: Ypsomed AG, 3401 Burgdorf (CH)
(72) Inventor: Schrul, Christian, 3400 Burgdorf (CH); Tschirren, Markus, 3400 Burgdorf (CH); Glauser, Oliver, 3008 Bern (CH); Loser, Peter, 3037 Herrenschwanden (CH)
(74) Representative: Kleiner, Stefan

(57) **Abstract**

The present invention relates to an injection device (1) for dispensing a drug from a reservoir (3) through a needle (5). The injection device (1) comprises a housing (50) defining a longitudinal axis, a drive member (30) movable relative to the housing (50) to dispense the drug, a spring member (6) operatively coupled the drive member (30) to move the drive member in a dispensing direction and a needle cover (10) movable along the longitudinal axis relative to the housing (50) between a needle covering position and a retracted position defining a needle cover movement along a first distance. Additionally, the spring member (6) is operatively coupled to the needle cover (10) such that the needle cover movement causes the spring member (6) to compresses or expand.

## Description

### FIELD OF THE INVENTION

The present invention relates to drug delivery devices or medicament delivery devices for injecting, delivering, administering, infusing or dispensing substances and/or liquids such as insulin, hormone preparations or vaccines. It departs from an injection device comprising a housing, a drive member movable relative to the housing, a spring member operatively coupled to the drive member to move the drive member in a dispensing direction and a needle cover movable along a longitudinal axis relative to the housing.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Also known is the use of autoinjectors with prefilled syringes. Autoinjectors usually comprise a body for housing the syringe as well as an automatic drive mechanism in order to move the plunger of the syringe upon actuation of the autoinjector. The drive mechanism of the autoinjector typically comprises a source of drive, such as a strong spring for moving a drive member, for example a rod, which acts on the plunger of the syringe. The prefilled syringe has a needle or cannula that is permanently attached to a first end of a syringe barrel or reservoir that is sealed at the opposing end by the plunger.

For safety and hygiene reasons it is desirable that the needle does not protrude from a housing of the autoinjector with the exception of the time when the needle is used for injection of a medicament. Thus, either the autoinjector moves the needle out of the housing for the injection and back into the housing after injection or the autoinjector provides a needle cover or needle guard which may be moved to unsheathe the needle for an injection and which may be moved back in a needle covering position after the injection.

The majority of autoinjectors are configured as single use devices which incorporate both the syringe and the drive mechanism in the same housing. Such devices are usually disposable for hygiene reasons.

In order to reduce costs and complexity it is desirable to design injection devices with a small number of parts and components.

WO11101379 A1 discloses an auto-injector with a single compression spring for advancing a syringe with a needle, injecting a dose of medicament and retracting the syringe and needle. Under load of the compression spring a syringe holder, a syringe carrier and the syringe are pushed forward while no load is exerted onto the piston inside the syringe. The injection needle is then inserted into an injection site. Subsequently, the compression spring moves the stopper in dispensing direction. When the stopper has nearly bottomed out in the syringe a decoupling member has reached an end position and it pushes against latches to decouple a retraction sleeve from the housing, so the retraction sleeve may slide away from the needle. The syringe holder is taken along in counter-dispensing direction by the retraction sleeve. Thus, the syringe and needle are retracted into a safe position inside the housing, e.g. into the initial position. The plunger is pulled back too.

WO09007305 A1 discloses an injection pen with an ampoule, an ampule holder and a piston rod operatively coupled to a drive spring. During the injection the drive spring drives the plunger rod in dispensing direction. When the piston rod is in an end position after the injection a driven nut changes the direction of rotation due to a reversed thread section. The reverse rotation of the nut causes the nut to move the ampoule holder in the proximal direction, whereby the needle is retracted from the tissue of the user.

In summary, these prior art approaches focus on a multiple use of the drive spring not only for an injection movement but also for a syringe retraction.

### DESCRIPTION OF THE INVENTION

It is an objective of the invention to provide an injection device with a reduced number of components.

This objective is achieved by the injection device according to the independent claims. Preferred embodiments are evident from the dependent claims.

The invention relates to an injection device for dispensing a liquid drug from a reservoir through a needle. The injection device comprises
- a housing, preferably a pen-shaped housing, defining a longitudinal axis;
- a drive member movable relative to the housing to dispense the drug from the reservoir;
- a spring member operatively (directly or indirectly) coupled the drive member to move the drive member in a dispensing direction to dispense the drug from the reservoir and
- a needle cover, preferably sleeve-shaped, movable along the longitudinal axis relative to the housing between a needle covering position (in which the needle is covered, i.e. not visible in a direction perpendicular to the longitudinal axis) and a retracted position (in which the needle is exposed) defining a needle cover movement along a first distance along the longitudinal axis.

The same spring member is further operatively (direct or indirectly) coupled to the needle cover such that the needle cover movement in a proximal or in a distal direction causes (or allows) the spring member to compress or expand.

That means the spring member is not only coupled to the drive member but also to the needle cover allowing to use a spring force of the spring member not only for a dispensing movement of the drive member but also for a needle cover movement, preferably to move the needle cover back into the needle covering position or/and to bias the needle cover when in the needle covering position.

Known device concepts either do not at all provide a needle cover member movable relative to a housing or require a separate cover spring to move or bias a needle cover member. In contrast to such approaches the injection device according to the invention does not require a separate cover spring member as the sole spring member is used both for a drive member dispensing movement and for a needle cover movement. This reduces the number of parts and in particular a second (metallic) spring member in the device is not required. This reduces complexity and manufacturing and assembly costs. Furthermore, the lower number of parts is advantageous in view of sustainability.

The injection device is preferably a disposable automatic injection device such as an autoinjector. Autoinjectors usually comprise a body for housing a reservoir in form of a syringe as well as an automatic drive mechanism to move a piston inside the reservoir upon actuation of the autoinjector.

The drive mechanism of the autoinjector typically comprises a compression spring or spiral spring for moving the drive member, for example a rod, which acts on the piston of the reservoir.

Alternatively, the injection device may be a hybrid device or semi reusable (or semi disposable) injection device comprising a disposable reservoir unit including the reservoir and preferably the needle cover and a reusable drive unit including the drive member.

The reservoir is preferably a prefilled syringe that has a needle or cannula permanently attached to a distal end of a syringe barrel or reservoir and that is sealed at the opposing end by a piston.

The spring member is operatively coupled to both the drive member and the needle cover. The term "operatively" means that the spring member may be coupled directly or indirectly via one or more intermediate member. For example, the spring member may be connected to a connector or intermediate member which in turn is connected to the drive member and/or to the needle cover, respectively. Such an intermediate member may be, for example, an axially movable sleeve or a rotation element rotatable relative to the housing.

The spring member preferably provides a spring force. By way of example, the spring member may be a metallic solenoid or spiral spring, a flat spring or leaf spring or an elastomer element or an elastically deformable plastic member adapted to provide a spring force in a biased state.

The needle cover is movable relative to the housing to cover or shield an injection needle. During injection when the injection needle is inserted into the injection site the needle cover is preferably in the retracted position. Before and/or after the injection the needle cover is preferably in the covering position to avoid unintentional access to the needle and to avoid injuries.

The drive member is adapted to move directly or indirectly the plunger or piston inside the reservoir in a dispensing direction to dispense the liquid drug from the reservoir. The drive member is preferably movable relative to the housing along the longitudinal axis, in particular non-rotatably. For that, the drive member may include splines or grooves adapted to engage corresponding grooves or splines in the housing to rotationally couple the drive member to the housing while allowing axial movement. The drive member may be, for example, a plunger rod, a piston or a drive sleeve.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the injection device carrying an injection needle or an injection cannula, whereas the term "proximal" is meant to refer to the opposite direction or end pointing away from the needle or cannula.

The term "injection device" or "injector" refers to a device that is removed from the injection site after each medication event or drug delivery process, whereas the term "infusion system" refers to a device with a cannula or needle that remains in the skin of the patient for a prolonged period of time, for example, several hours.

The drive member is preferably a plunger rod which is biased in a dispensing direction before a start of the injection by the spring member. Upon release or activation of the injection device the plunger rod may be driven by the spring force of the spring member in dispensing direction. The plunger rod may be adapted to carry out a rotational, a helical or a linear movement during dispensing. Alternatively, the drive member may be a drive sleeve or a rotating element providing a dispensing movement. The drive spring bias prior to dispense or prior activation may be blocked by the intermediate element.

If the needle cover is moved towards the retracted position the spring member is preferably compressed while the spring member maintains the plunger rod in a biased state before starting to drive the plunger rod in dispensing direction. That means a first portion or end of the spring member is used to bias the needle cover in the covering position and a second portion or end of the spring is used to bias the plunger rod in a dispensing direction, with an intermediate portion of the spring member being fixed, or being a mere connecting element. Alternatively, the whole spring member provides the needle cover bias and the plunger rod bias.

In a preferred embodiment the plunger rod is hollow and the drive member is a compression spring at least partially arranged inside the hollow plunger rod. This provides for a space-saving design. Alternatively, the spring may be arranged proximally of the plunger rod.

The operative coupling of the spring member to the needle cover is preferably provided by a rotation member rotatable relative to the housing and preferably axially fixed relative to the housing. The rotation member is adapted to transfer, by a rotational movement of the rotation member, the needle cover movement into an axial spring member compression movement. The spring compression movement is along the longitudinal axis and is preferably devoid of any rotational movement components. The spring compression movement may be carried out, for example, by a supporting element or spring connector element.

Thus, if the needle cover is moved from the covering position into the retracted position along the first distance the rotation member is rotated. This rotation in turn generates a spring member compression movement causing the spring member to compress. That means that a spring force (introduced by the spring compression movement) has to be overcome to move the needle cover towards the retracted position.

The rotation member thus couples the needle cover to the spring member allowing a spring force to be transferred to the rotation member for biasing the needle cover, preferably into the covering position. The rotation member is preferably rotatable around the longitudinal axis relative to the housing.

Alternatively, the spring member may be directly coupled to the needle cover or the spring member may be coupled to the needle cover by another intermediate member, for example, an axially movable coupling member.

In a preferred embodiment the above described spring compression movement defines a second distance wherein the rotation member provides a transmission ratio (gearing ratio) such that the second distance is shorter than the first distance (which is the movement distance of the needle cover between the covering position and the retracted position). Due to the transmission ratio a relatively long needle cover movement can be transferred into a shorter spring compression movement. That allows to apply a suitable spring force or to adjust the spring force used for biasing the needle cover. Furthermore, the transmission ratio allows to optimize the movement length and thus provides for a space-saving design.

The transmission ratio may be implemented, for example, by a cam control, by a transmission by means of lever arms or cantilevers of different lengths or by a toggle joint.

The injection device preferably includes a cam control adapted to transfer a needle cover movement in a proximal direction along the longitudinal axis into the spring compression movement in a distal direction along the longitudinal axis. The cam control thus reverses or inverts an axial needle cover movement into an axial spring compression movement in a counter direction. This allows to reliably compress the spring member and thus to bias the needle cover into the covering position while still providing a spring force in dispensing direction for moving or biasing the drive member. Hence, the spring member may be strained or pre-strained upon device assembly in order to move the drive member in a distal direction, and provided with additional strain by the needle cover movement on a translated or transmitted a distal movement of the proximal spring end.

The cam control may be implemented, for example, by means of a cam, ram or protrusion guided in a spline groove, a nut or a guide rail.

The injection device comprises preferably a spring connector adapted to move along the longitudinal axis and coupled to the rotation member and further coupled to the spring member to provide the spring compression movement. Hence, the spring connector couples the rotation member and the spring member. The spring connector is preferably arranged at an end portion or at a terminal end of the spring, preferably of a spiral spring and thus allows a reliable compression of the spring if a force from the rotation member is transferred to the spring connector. For example, the spring connector may have a cap shape or it may be sleeve-shaped. Alternatively, the spring can be connected directly to the rotational member without a spring connector or any other intermediate member.

In a preferred embodiment the needle cover is coupled to the rotation member by a first cam guided in a first cam groove and the spring connector is coupled to the rotation member by a second cam guided in a second cam groove, wherein the rotation member is rotatable around the longitudinal axis.

The first and second cam groove may be arranged on the rotation member and the first cam may be arranged on the needle cover and the second cam may be arranged on the spring connector. The two cam grooves may be arranged on the rotation member at suitable longitudinal locations, even at approximately the same longitudinal position, rotated with respect to each other by 180° or 90°. Alternatively, the first and second groove may be arranged on the needle cover and the spring connector respectively and correspondingly, the first and second cam may be arranged on the rotation member.

During the needle cover movement the first and second cam travel in the first and second cam groove thereby transferring the axial movement of the needle cover in a rotational movement of the rotation member and further back into an axial movement of the spring connector. The latter provides the spring compression movement which is preferably in counter direction to the needle cover movement.

Therefore, the rotation member allows to reliably transfer forces and movements by the first and second cam and groove engagement between the needle cover and the spring or the spring connector.

In order to transfer a movement along the longitudinal axis of the needle cover or of the spring connecter into a rotational movement of the rotation member the first cam groove comprises preferably a groove section or groove portion angled to the longitudinal axis in a first angle and the second cam groove preferably comprises a groove section or groove portion angled to the longitudinal axis in a second angle which is larger or smaller than the first angle.

In case the second angle is larger than the first angle an axial component of the travel path of the second cam is shorter than an axial travel path component of the first cam. Hence, the first distance of the needle cover can be transferred into a shorter second distance of the spring connector. This allows for an optimal length and space-saving design of the injection device.

As an alternative the second angle may be smaller and thus the axial component of the second cam may be longer such that the second distance is longer than the first distance.

In a second embodiment of the present invention the rotational member may be a cantilever rotatable around an axis perpendicular to the longitudinal axis of the housing. Thus, in the second embodiment the rotation member does not include any cam control. A first half or a first end portion of the cantilever is operatively coupled to the needle cover and a second half or second end portion of the cantilever is operatively coupled to the spring member or to the spring connector, if any. A needle cover movement is thus transferred to the spring member and vice versa by the cantilever.

The needle cover and the spring member can be coupled directly or indirectly via intermediate member to the cantilever. The cantilever may have a shape of a rod or elongated member. A transmission ratio may be provided by the cantilever depending on the position of a pivot point or the position of the connections between the cantilever and needle cover or the spring member respectively. The spring member is preferably connected to the cantilever via a spring connector. The spring connecter may be arranged at an end portion of the spring as described above.

In a third embodiment of the present invention the rotational member may be a toothed wheel rotatable around an axis perpendicular to the longitudinal axis of the housing. The needle cover and the spring member comprise each a toothed rack engaging the toothed wheel such that a movement of the needle cover is transferred to a spring compression movement and vice versa. As described with respect to the first and second embodiment the spring member may be connected to a spring connector which provides the toothed rack and thus the spring is connected to the toothed wheel via spring connector.

A transmission ratio may be provided by the toothed wheel and rack engagement depending on the size and number of the teeth. The racks of the needle cover and the spring member (or spring connector) are preferably linear racks extending along the longitudinal axis.

The toothed wheel has preferably more than one gearwheel to provide a transmission ration between the needle cover and the spring member. Particularly, the toothed wheel may comprise a first gear wheel with a first outer diameter engaging the needle cover rack and a second gear wheel with a second outer diameter different than the first outer diameter engaging the spring rack. Depending on the size of the first and second diameter a particular transmission ratio can be achieved.

In a fourth embodiment of the present invention the injection device includes a spring member having different outer spring diameters. Hence, in this embodiment the injection device does not include a rotation member. The spring member comprises preferably a first portion coupled to the drive member and a proximal portion coupled to the needle cover. The first portion has a first outer diameter and the second portion has a second outer diameter different from the first outer diameter, wherein the spring member is made of a continuous spring winding connecting the first and second portion. That means the spring member is made of one single piece of wire and thus there is no need to connect two separate springs.

The first portion is preferably a distal portion and the second portion is a proximal portion of the spring member. The first and second portion may be arranged coaxially to each other. The two portions are wound by one single and continuous wire.

As the spring member has different diameters the spring force can be adjusted and fitted to the appliance. For example, the first portion may have a smaller diameter for driving the drive member and to fit inside a hollow plunger rod. The second portion may have a diameter larger than diameter in the first portion for biasing the needle cover into the covering position.

Instead or additionally, the spring member may include different spring coil pitches. Hence, a first portion may have a different spring coil pitch than a second portion of the spring member.

The injection device is preferably an autoinjector comprising a syringe holder adapted to fixedly hold a prefilled syringe relative to the housing. That means the syringe is not movable relative to the housing during the complete injection process. Thus, the user has to insert manually the injection needle into the injection site.

Autoinjectors are typically designed to administer the whole content of the prefilled syringe or the complete dose in one single injection stroke. The autoinjector is thus preferably devoid of any dose setting or dose correction capabilities.

The injection device may further comprise a drive mechanism comprising the drive member in form of a plunger rod. The drive mechanism is adapted to move the plunger rod in dispensing direction.

The plunger rod comprises a first plunger rod member and a second plunger rod member, wherein the first and second plunger rod member are movable to each other along the longitudinal axis.

The first plunger rod member includes a cone having a minimum diameter at a tip of the cone and a maximum diameter at a basis of the cone. The second plunger rod member includes an opening with diameter larger than the minimum and smaller than maximum diameter of the cone, preferably the material or an deflectable portion of the first plunger rod member and/or the second plunger rod member is deformable such that the cone and/or the opening can be deformed.

During a plunger rod dispensing movement the cone is moved relative to the opening such that the cone is (further) inserted into the opening such that outer surface of the cone gets into contact with inner surface of the opening. Thereby the opening is widen or/and the cone is deformed creating a resistance force which decelerates the movement of the first plunger rod member relative to the second plunger rod member.

That means during a dispensing movement, when the piston rod is moved distally by a drive spring force, a distal end (e.g. the first plunger rod member) of the plunger hits a piston inside the reservoir. Due to the immediate stop the reservoir and other components of the injection device have to absorb impact energy from the driven plunger rod.

According to the present embodiment the cone is (further) moved or pressed into the opening when the plunger rod first hits the piston of the reservoir. As the opening is smaller than the cone basis the cone may press against a rim portion of the opening. Thereby, the cone and/or the opening (in particular the rim portion) may deform and can thus absorb energy from the moving plunger rod. This is advantageous as a decelerated movement of the plunger rod may prevent breakage or damage of the reservoir or syringe when the plunger rod first hits the piston of the reservoir.

In a preferred embodiment the first plunger rod member is a distal part (plunger rod tip) and the second plunger rod member is the rest or proximal part of the plunger rod. The first member may include a distal contact surface adapted to abut and move a piston inside the reservoir. The second plunger rod member may form a hollow piston rod providing a cavity adapted to accommodate the drive spring.

The cone basis is preferably arranged on the first plunger rod member and the cone tip is preferably located at a proximal end of the first plunger rod member. Accordingly, the second plunger rod member includes the opening at a distal end of the second plunger rod member. The cone may be at least partially arranged inside the opening in an initial state or shipping state. During use and in particular when the plunger rod hits the piston the cone is further inserted into the opening to absorb energy as described above.

### BRIEF DESCRIPTION OF THE DRAWINGS

The subject matter of the invention will be explained in more detail in the following text with reference to preferred exemplary embodiments which are illustrated in the attached drawings, in which:
- Fig. 1a: depicts a sectional view of an autoinjector according to the invention in an initial state,
- Fig. 1b: depicts the autoinjector with the needle cover in the retracted position before start of the injection,
- Fig. 1c: depicts the autoinjector after the injection with the needle cover in the covering position;
- Fig. 2a-2c: depict schematical views of a second embodiment of the invention with an autoinjector comprising a cantilever;
- Fig. 3a-3c: depict schematical views of a third embodiment of the invention with an autoinjector comprising a toothed wheel;
- Fig. 4: depicts a sectional view of a fourth embodiment of the invention with an autoinjector with a compression spring;
- Fig. 5: depicts a perspective view of the compression spring of the autoinjector shown in figure 4 and
- Fig. 6: depicts a sectional view of the plunger rod and a portion of the syringe of the autoinjector and
- Fig. 7: depicts a perspective view of the plunger rod and syringe wherein the rest of the autoinjector is not shown.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

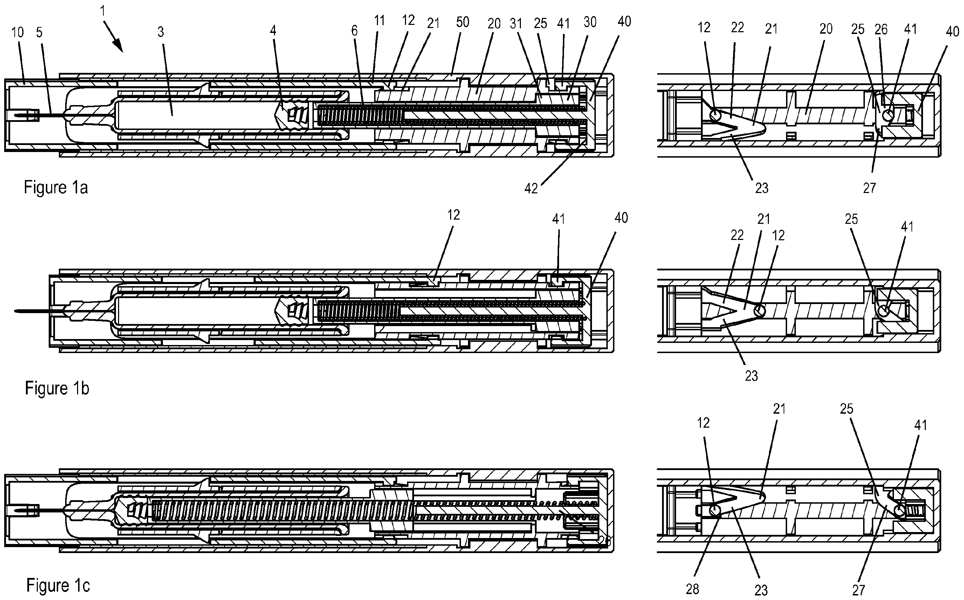

In the following the term "distal" refers to the side of the device where an injection needle is located (e.g. left hand side in figures 1 to 5). The term "proximal" refers to the opposite side which is furthest away from the needle (right hand side in figures 1 to 5).

Figures 1a to 1c depict sectional views of an injection device according to the present invention. In the left sectional view the cut for the sectional view runs along the longitudinal axis of the injection device and in the right sectional view the cut runs through a plane parallel and offset to the longitudinal axis. Furthermore, the right sectional view is 90° rotated compared to the left sectional view of figures 1a to 1c.

In the shown embodiment the injection device is a disposable autoinjector 1 including a prefilled syringe 3 with a permanently attached injection needle 5. The autoinjector 1 comprises a housing 50 which holds or supports the prefilled syringe 3 non-movable inside the housing, a hollow plunger rod 30, a drive spring 6 in form of a compression spring, a needle cover sleeve 10 axially movable relative to the housing 50, a rotation sleeve 20 and a spring connector 40 arranged at a proximal end of the spring 6.

Figure 1a shows the autoinjector 1 in an initial or shipping state. The needle cover sleeve 10 is in a distal covering position in which the injection needle 5 is covered or shielded. Furthermore, in the initial state a rigid needle shield RNS (not shown) is mounted onto the injection needle 5. Furthermore, a cap remover is mounted onto the RNS. The needle cover sleeve 10 is coupled to the rotation sleeve 20 by distal cam control. That is, the needle cover sleeve 10 includes an axially extending arm 11 comprising a cam 12 on its proximal end. The cam 12 is guided in a distal groove 21 arranged on a distal portion of the rotation sleeve 20. As best shown in the right view of figure 1a the distal groove 21 is V-shaped. That means the groove comprises a first groove section 22 and second groove section 23 each running in an acute angel (for example about 20 to 40° to the longitudinal axis). As described below the cover sleeve cam 12 is guided within the distal groove 21 during a movement of the needle cover sleeve 10.

The rotation sleeve 20 is further coupled to the spring connector 40 by a proximal cam control. For that purpose, the rotation sleeve 20 includes on a proximal portion a proximal groove 25 guiding a cam 41 of the spring connector. Also the proximal groove 25 includes a first groove section 26 and a second groove section 27 running angled to the longitudinal axis and being V-shaped. Compared to the groove sections 22, 23 of the distal groove 21 the proximal groove sections 26, 27 are in a flatter angle of about 60° to the longitudinal axis.

The spring connector 40 supports the compression spring 6. That means the spring 6 abuts on its proximal end on an end surface 42 of the spring connecter 40 and on its distal end the spring 6 abuts an inner surface of the hollow plunger rod 30 and thereby biases the plunger rod 30 in a distal dispensing direction. In the initial state the plunger rod 30 is held in position as a radial protrusion 31 of the plunger rod abuts a stop surface on an inside of the rotation sleeve 20, which is coaxially arranged to the plunger rod 30. The rotation sleeve 20 is axially fixed relative to the housing 50.

In the following the function of the injection process with the autoinjector 1 is described in detail.

In a first step the user removes a distal cap (not shown) and thereby removes the RNS from the injection needle 5. In order to start the injection the user places the distal end of the cover sleeve 10 onto the injection site and in a next step pushes the autoinjector 1 towards the skin. Thereby the needle covers sleeve 10 is moved axially into the housing 50 form a covering position into a retracted position. The arm 11 and its cam 12 are thus axially shifted in proximal direction relative to the rotation sleeve 20. As the cam 12 is guided inside the distal groove 21 and as the arm 11 is linearly guided by a guide of the housing 50 the cam 12 cannot deflect laterally but presses on a lateral side of the first angled groove section 22. As the rotation sleeve 20 is axially fixed relative to the housing 50 the rotation sleeve 20 starts to rotate due to the force from the cam 12. As the needle cover sleeve 10 is further pushed in proximal direction towards the retracted position the cam 12 travels inside the first groove section 22 keeping the rotation sleeve 20 rotated.

As the cam 41 of the spring connector 40 is guided in the proximal groove 25 the rotating rotation sleeve 20 brings a lateral wall of the first groove section 26 of the proximal groove 25 in contact with the cam 41 of the spring connector 40. As the spring connector 40 is rotationally fixed but axially movable relative to the housing 50 the spring connector 40 is forced to move distally because the angled groove section 26 of the rotating rotation sleeve presses the spring connector cam 41 distally. The distal movement of the spring connector 40 leads to a compression of the spring 6 at least in a proximal portion of the spring. That means due to the compression a spring force has to be overcome to move the needle cover sleeve 10 towards the retracted position.

In the state shown in figure 1b the needle cover sleeve is in its retracted position (after pressing the autoinjector onto the injection site) and the rotation sleeve 20 is rotated such that the radial protrusion 31 of the plunger rod 30 is out of engagement with the rotation sleeve stop surface. As a consequence the plunger rod 30 is free to move axially. Hence, the compressed spring 6 can expand such that the spring force moves the plunger rod 30 distally in dispensing direction to move the piston 4 inside the syringe 3 distally and thus the medication inside the syringe 3 is dispensed.

In an end of the injection state as shown in figure 1b the needle cover sleeve 10 is in its proximal retracted position and the spring connector 40 is in a distal end position, best shown in the right view of figure 1b. As shown the cam 12 of the needle cover 10 is located in the most proximal position, namely in a juncture of the first groove section 22 and second groove section 23 and the cam 41 of the spring connector 40 is located in the most distal position, namely in a juncture of the first groove section 26 and second groove section 27.

When the injection is completed the user removes the autoinjector 1 from the injection site. As the compression spring 6 is partially compressed by spring connector 40 (being in its distal end position) the needle cover sleeve 10 is biased distally towards the covering position by the spring force due to the cam control. As the autoinjector 1 is no longer pressed against the injection site and thus the spring can expand the cam 12 of the needle cover sleeve 10 is no longer pressed into its proximal end position as shown in figure 1b.

The spring force presses the spring connector 40 proximally and thereby the cam 41 of the spring connector presses against a proximal wall of the second groove section 27. This causes the rotation sleeve 20 to further rotate in rotation direction such that the cam 41 completely enters into the second groove section 27 of the proximal groove 25. The rotating rotation sleeve 20 causes the cover sleeve cam 12 to enter into the second groove section 23 of the distal groove 21 (see right hand view in figure 1c) thereby allowing the needle cover sleeve 10 to move distally towards the covering position.

Figure 1c depicts the autoinjector 1 in a used state after removal from the injection site. In this state the needle cover sleeve 10 is back in its covering position and locked to prevent reuse. For this purpose, the second groove section 23 of the distal groove 21 includes a ledge 28 as shown in the right sectional view of figure 1c. The cam 12 would be pressed against the ledge 28 in case a proximal force acts on the needle cover sleeve 10. Thereby a retraction of the cover sleeve 10 after the injection is prevented.

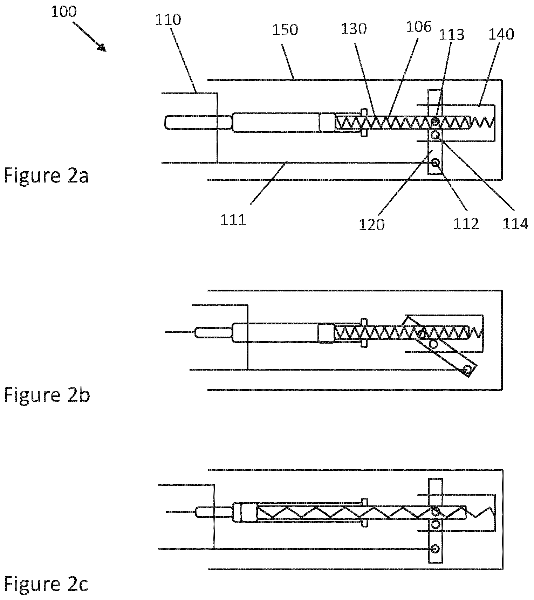

Figures 2a to 2c depict schematic views of a second embodiment of an autoinjector 100 according to the invention.

Figure 2a depicts the autoinjector 100 in an initial or shipping state. The needle cover sleeve 110 includes an arm 111 rotatably coupled to a first end of a cantilever 120 at a first pivot 112. The cantilever is rotatable relative to the housing 150 around an axis perpendicular to the longitudinal axis at a central pivot 114. On a second end of the cantilever 120 the the spring connector is rotatably coupled to the cantilever 120 at a second pivot 113. As shown in figure 2a in the initial state the cantilever 120 is in an upright position.

If the needle cover sleeve 110 is retracted from its covering position towards its retracted position as shown in figure 2b the proximally moving needle cover arm 111 rotates (counterclockwise in the view of figures 2b) the cantilever 120 around its central pivot 114. This causes the second end of the cantilever 120 (with second pivot 113) and with the spring connector 140 to move distally and thereby the spring 106 is partially compressed. That means a spring force has to overcome when moving the needle cover sleeve 110 proximally.

The autoinjector 100 can now be fired and the drive spring 106 expands to move the plunger rod 130 distally. After the injection the autoinjector 100 is removed from the injection site and the needle cover sleeve 110 is moved back into its covering position by the spring force as the spring 106 can partially expand and thus the spring connector 140 is moved proximally and the cantilever 120 is rotated in counter direction (clockwise in the figures).

Figure 2c shows a state after the injection where the drive spring 106 is expanded and the plunger rod 130 is in a distal end position. The needle cover sleeve 110 is back in the covering position and accordingly the cantilever 120 is back in the upright position. Depending on the location of the pivots 112, 113 and 114 a transmission ratio can be provided, for example to transfer a needle cover movement distance into a shorter spring compression movement distance.

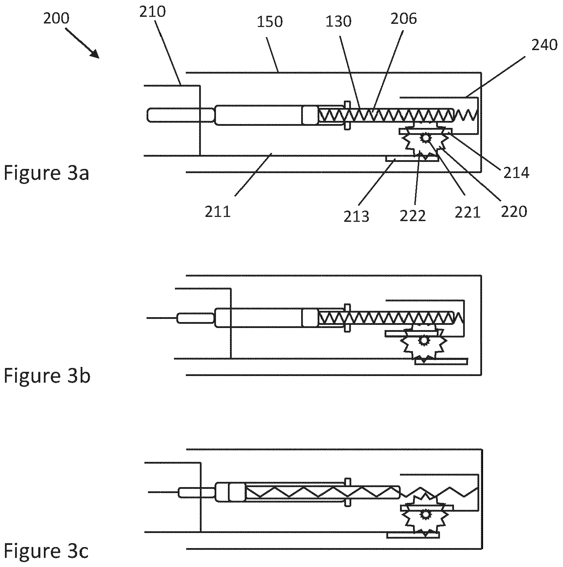

Figures 3a to 3c depict a third embodiment of the autoinjector 200 according to the invention. In contrast to the second embodiment the autoinjector 200 according to the third embodiment includes a toothed wheel 220 comprising a small gearwheel 221 and a large gearwheel 222 which are rotatable fixed to each other. The arm 211 of the needle cover sleeve 210 includes a corresponding toothed rack 213 extending along the longitudinal axis and engaging the large gearwheel 222. The spring connector 240 includes a corresponding toothed rack 214 engaging the small gearwheel 221.

Figure 3a shows the autoinjector 200 in an initial state or shipping state. The toothed wheel 220 is located in-between the two toothed racks 213, 214. When the needle cover sleeve 210 is moved into its retracted position the proximally moving rack 213 rotates the toothed wheel 220. As the rack 214 of the spring connector 240 is in toothed engagement with the small gearwheel 221 the rotating toothed wheel 220 causes the spring connector rack 214 to move in distal direction. The different diameters of the gear wheels 221, 222 provide a transmission ratio. The rotating toothed wheel moves the spring connector 240 distally and the spring 206 is compressed and thus exerts a force against a proximal cover sleeve movement.

Figure 3b shows an end of injection state where the needle cover sleeve 210 is in the retracted position and the spring connector 240 is in the distal end position thereby compressing the spring 206 at least in a proximal portion of the spring.

After removal of the of the autoinjector 200 from the injection site the compressed drive spring 206 can expand and moves the spring connector 240 in proximal direction. This causes the toothed wheel 220 to rotate back and thus moves the rack 213 and the needle cover sleeve 210 into its covering position. Figure 3c shows the end state after the injection with the needle cover sleeve back in the covering position.

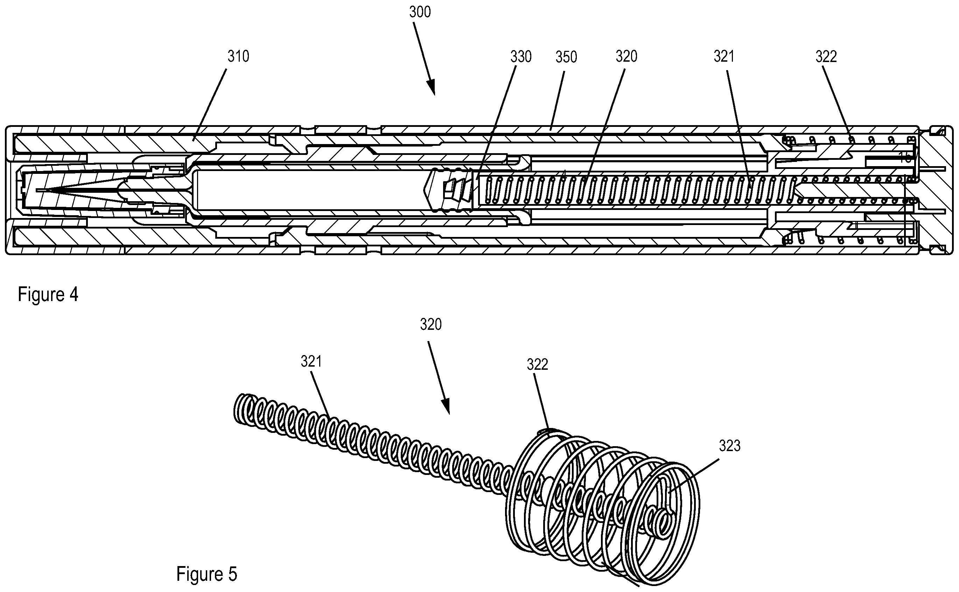

Figures 4 and 5 depict a fourth embodiment of the present invention. In contrast to the previous embodiments the autoinjector 300 does not include any rotation sleeve. Instead, the autoinjector 300 according to the fourth embodiment comprises one single spiral spring 320 featuring two different spring portions arranged inside the housing 350. A distal spring portion 321 has a smaller diameter and a proximal spring portion 322 has a larger diameter which is outside and coaxially arranged to the distal portion 321. That means in respect to the length the proximal spring portion 322 overlaps a part of the length of the distal spring portion 321 best shown in the perspective view of the spring 320 in figure 5. The spring 320 is made of one single piece (monolithic) and hence one continuous wire connects the distal and proximal portion 321, 322.

As shown in figure 4 the distal spring portion 321 is arranged inside the hollow plunger rod 330 and biases the plunger rod in distal dispensing direction. The proximal portion 322 is connected to the needle cover sleeve 310 biasing the needle cover sleeve into its covering position. In this way the spring 320 provides two functions with one wire: it provides the drive force for the plunger rod 330 and it provides a force to bias the needle cover sleeve 310 in the covering position.

As an alternative to the above described first to fourth embodiments the spring for the needle cover sleeve may be an elastically deflectable arm extending along the longitudinal axis and preferably made of plastic. In this embodiment the plunger rod is biased and driven by a metallic compression spring. The deflectable arm may bias the needle cover sleeve into the covering position as the arm is slightly deflected in an initial position. If the needle cover sleeve is moved proximally into the retracted position the arm is laterally deflected (away from the longitudinal axis). As the arm is elastically deformed after removal of the autoinjector from the injection site the arm can move back into its initial position and thus moves the cover sleeve back towards the covering position. The arm may be integrally formed in an end cap of the autoinjetor. The end cap is fixedly attached to an autoinjector housing.

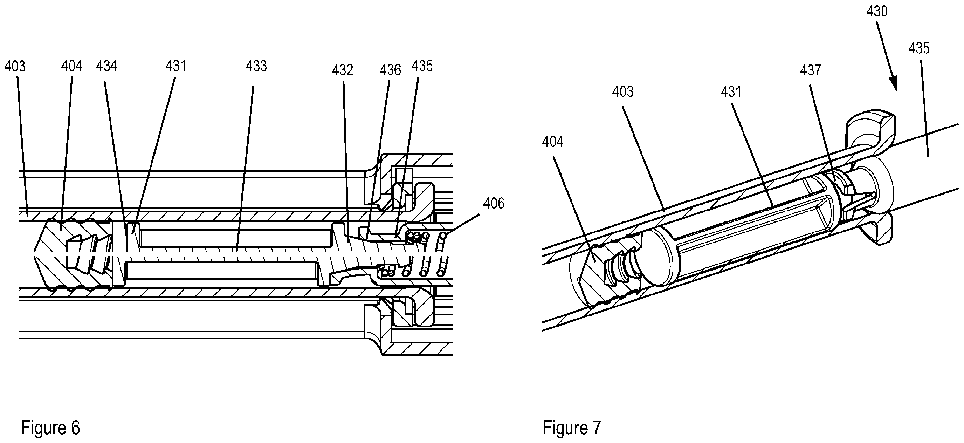

Figure 6 depicts a sectional view of a plunger rod 430 of the autoinjector. The upper part of the view the plunger rod is not yet in contact with the piston 404 inside the syringe 403 and in the lower part of the view the plunger rod 430 has hit and abuts the piston 404.

Figure 7 depicts a perspective view of the plunger rod 430 in the position as shown in the upper part of figure 6.

The plunger rod 430 comprises a distal part 431 including a cone 432 and a proximal part 435 including an opening 436 and a hollow plunger rod body, which accommodates the compression spring 406 in an inner cavity.

The distal part 431 or plunger rod tip provides the cone 432 wherein a cone basis is located on a distal side and a part of the cone and in particular the tip of the cone 432 is inserted into the opening 436 of the proximal part 435. Thus, the proximal part 435 comprises the opening 436 which connects the outside and the cavity.

When the autoinjector is fired the plunger rod 430 is moved by the drive spring 406 in distal dispensing direction. When a distal contact surface 434 first hits the piston 404 of the syringe 403 the proximal part 435 is moved relative to the distal part 431 and the cone 432 is further moved into the opening or cavity of the proximal part 435 thereby deforming an entrance area or outer rim portion 437 of the opening 436. The rim portion 437 is interrupted by a V-shaped recess (see figure 7) which weakens the entrance area to facilitate the deformation. Therefore, energy from the moving plunger rod 430 can be absorbed by the cone 432 and opening 436 interaction.

The distal part 431 may include an elongated section 433 as shown in figures 6 and 7 to compensate for partially filled syringe. That means the same syringe size can be used for different dose sizes as the distal part 431 compensates with its elongated section 433 the length (or volume) not used with a partially filled syringe. However, the elongates section 433 is not mandatory and its length may depend on the syringe used. In any case, the proximal part 435 has the same length independent of the length of the section 433.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | |
|---|---|---|---|
| 1 | autoinjector | 200 | autoinjector |
| 3 | syringe | 206 | spring |
| 4 | piston | 210 | needle cover sleeve |
| 5 | needle | 211 | arm |
| 6 | drive spring | 213 | toothed rack cover sleeve |
| 10 | needle cover sleeve | 214 | toothed rack spring connector |
| 11 | arm | 220 | toothed wheel |
| 12 | cam | 221 | small gearwheel |
| 20 | rotation sleeve | 222 | large gearwheel |
| 21 | distal groove | 240 | spring connector |
| 22 | first distal groove section | | |
| 23 | second distal groove section | 300 | autoinjector |
| 24 | radial guide | 310 | needle cover sleeve |
| 25 | proximal groove | 320 | spring |
| 26 | first groove section | 321 | distal spring portion |
| 27 | second groove section | 322 | proximal spring portion |
| 28 | ledge | 330 | plunger rod |
| 30 | plunger rod | 350 | housing |
| 31 | radial protrusion | | |
| 40 | spring connector | 403 | syringe |
| 41 | cam | 404 | piston |
| 42 | end surface | 406 | drive spring |
| 50 | housing | 430 | plunger rod |
| 51 | guide groove | 431 | distal plunger rod member |
| 100 | autoinjector | 432 | cone |
| 106 | spring | 433 | elongated section |
| 110 | needle cover sleeve | 434 | contact surface |
| 111 | arm | 435 | proximal plunger rod member |
| 112 | first pivot | 436 | opening |
| 113 | second pivot | 437 | rim portion |
| 114 | central pivot | | |
| 120 | cantilever | | |
| 130 | plunger rod | | |
| 140 | spring connector | | |
| 150 | housing | | |

## Claims

1. An injection device (1) for dispensing a drug from a reservoir (3) through a needle (5), the injection device (1) comprising
- a housing (50) defining a longitudinal axis;
- a drive member (30) movable relative to the housing (50) to dispense the drug;
- a spring member (6) operatively coupled the drive member (30) to move the drive member in a dispensing direction and
- a needle cover (10) movable along the longitudinal axis relative to the housing (50) between a needle covering position and a retracted position defining a needle cover movement along a first distance,
**characterized in that** the spring member (6) is additionally and operatively coupled to the needle cover (10) such that the needle cover movement causes the spring member (6) to compresses or expand.

2. Injection device (1) according to claim 1, wherein the drive member (30) is a plunger rod which is biased by the spring member (6) before an injection.

3. Injection device (1) according to claim 1 or 2, wherein a movement of the needle cover (10) towards the retracted position causes the spring member (6) to compress while maintaining the plunger rod bias.

4. Injection device (1) according to claim 2 or 3, wherein the plunger rod (30) is hollow and the spring member (6) is a compression spring at least partially arranged inside the hollow plunger rod (30).

5. Injection device (1) according to any of claims 1 to 4, wherein the operative coupling of the spring member (6) to the needle cover (10) is provided by a rotation member (20) rotatable relative to the housing (50), wherein the rotation member (20) is adapted to transfer, by a rotational movement of the rotation member (20), the needle cover movement into an axial spring member compression movement.

6. Injection device (1) according to claim 5, wherein the spring compression movement defines a second distance wherein the rotation member (20) provides a transmission ratio such that the second distance is shorter than the first distance.

7. Injection device (1) according to any of claims 5 to 7, wherein the rotation member (20) includes a cam control (21, 25) adapted transfer a needle cover movement in proximal direction into the spring member compression movement in a distal direction.

8. Injection device (1) according to any of claims 5 to 7, further comprising a spring connector (40) adapted to move along the longitudinal axis and coupled to the rotation member (20) and further coupled to the spring member (6).

9. Injection device (1) according to claim 8, wherein the needle cover (10) is coupled to the rotation member (20) by a first cam (12) guided in a first cam groove (21) of the rotation member and the spring connector (40) is coupled to the rotation member (20) by a second cam (41) guided in a second cam groove (25) of the rotation member, wherein the rotation member (20) is rotatable around the longitudinal axis.

10. Injection device (1) according to claim 9, wherein the first cam groove (21) is angled to the longitudinal axis in a first angle and the second cam groove (25) is angled to the longitudinal axis in a second angle which is different than the first angle.

11. Injection device (100) according to any of claims 1 to 6, wherein the rotational member is a cantilever (120) rotatable around an axis perpendicular to the longitudinal axis of the housing (150), wherein a first end portion of the cantilever (120) is coupled to the needle cover (110) and a second end portion of the cantilever (120) is operatively coupled to the spring member (106).

12. Injection device (200) according to any of claims 1 to 6, wherein the rotational member is a toothed wheel (220) rotatable around an axis perpendicular to the longitudinal axis of the housing, wherein the needle cover (210) and the spring connector (240) each comprise a toothed rack (213, 214) engaging the toothed wheel (220).

13. Injection device (200) according to claim 12, wherein the toothed wheel (220) includes a first gearwheel (221) with a fist diameter and a second gearwheel (222) with a second diameter different than the first diameter.

14. Injection device (300) according to any of claim 1 to 4, wherein the spring member (320) comprises a first portion (321) coupled to the drive member (330) and a second portion (322) coupled to the needle cover (310) wherein the first portion (321) has a first outer diameter and the second portion (322) has a second outer diameter different from the first outer diameter, wherein the spring member (320) is made of a continuous spring winding connecting the first and second portion (321, 322).

15. Injection device (1) according to any of claims 1 to 14, wherein the injection device is an autoinjector with a housing (50) and the reservoir (3) is a prefilled syringe fixedly hold inside the housing (50).
